(19) Europäisches Patentamt
European Patent Office
Office européen des brevets

(11) Publication number: **0 025 638**
**B1**

(12) **EUROPEAN PATENT SPECIFICATION**

(45) Date of publication of patent specification: **11.05.83**

(21) Application number: **80302619.4**

(22) Date of filing: **31.07.80**

(51) Int. Cl.³: **C 07 C 175/00,**
C 07 C 49/557,
C 07 C 69/007,
C 07 C 69/013 //A23L1/226,
A61K7/00, A24B3/12,
C11D3/50, C11D9/44

(54) **Process for the production of 1-crotonoyl-2,6,6-trimethylcyclohexa-1,3-diene and esters of alkynylcyclohexane derivatives as intermediates therefor.**

(30) Priority: **01.08.79 GB 7926767**

(43) Date of publication of application:
**25.03.81 Bulletin 81/12**

(45) Publication of the grant of the patent:
**11.05.83 Bulletin 83/19**

(84) Designated Contracting States:
**BE CH DE FR GB IT LI NL**

(56) References cited:
**CH - A - 548 967**
**CH - A - 563 326**
**DE - A - 2 804 597**
**DE - B - 2 242 751**

(73) Proprietor: **PFW (Nederland) B.V.**
**Nijverheidsweg Zuid 7**
**NL-3812 EA Amersfoort (NL)**

(72) Inventor: **Hulshof, Lumbertus Albregt**
**Seringenlaan 5**
**Hoevelaken (NL)**

(74) Representative: **De Minvielle-Devaux, Ian Benedict Peter et al,**
**CARPMAELS & RANSFORD 43, Bloomsbury Square**
**London WC1A 2RA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

**EP 0 025 638 B1**

# Process for the production of 1-crotonoyl-2,6,6-trimethylcyclohexa-1,3-diene and esters of alkynylcyclohexane derivatives as intermediates therefor

The present invention relates to a novel process for preparing 1-crotonoyl-2,6,6-trimethylcyclohexa-1,3-diene having the formula:

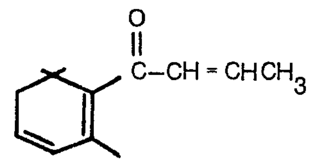

I

The invention further relates to novel starting materials used in the said process.

The compound of formula I, 1-crotonoyl-2,6,6-trimethylcyclohexa-1,3-diene, is a highly odoriferous compound found in nature as a trace component in several fruits such as, e.g., raspberry, apple and grapes, and several plant species such as deer tongue, tobacco, peppermint and Bulgarian rose, and in beer, wines, and rum. Its organoleptic and olfactory properties make it useful as a component in aromas and perfume compositions and as an aroma-additive to foodstuffs, beverages, pharmaceuticals and tobacco products. It is also used in the preparation of synthetic essential oils such as mock-orange and rose oil.

Because of the growing industrial importance of the compound and its limited availability from natural sources, much attention has been directed to the synthesis of 1-crotonoyl-2,6,6-trimethylcyclohexa-1,3-diene and to the structurally related compounds which are also of industrial interest. The interest in the compound is demonstrated by the numerous methods of preparation which have been reported, e.g., German patent applications 1,807,568, 2,022,216, 2,065,322, 2,065,323, 2,065,324, 2,240,311, 2,242,751, 2,244,680 and 2,305,140; Dutch patent application 75.10914; U.S. patent 3,928,456; Swiss patents 563,326 and 548,967; French patent 2,174,306; Japanese patent applications 74.75556 (Chem. Abstr. *82*, 124888 (1975) and 75.69048 (Chem Abstr. *84*, 30532 (1976), Helv. Chim. Acta *53*, 541 (1970), ibid. *54*, 1767, 1899 (1971), ibid. *56*, 310, 1503, 1514 (1973), J.C.S. Chem. Commun. *1973*, 161 and J.C.S. Perkin Trans. I, *1975*, 1727.

Most of the synthetic methods described in these references are laboratory methods which are not readily adaptable to profitable commercial use.

It is the object of the present invention to provide a method of preparing crotonoyl-2,6,6-trimethylcyclohexa-1,3-diene in a technically simple and commercially feasible manner which suppresses the yield of undesirable by-products.

The process of the invention comprises treating an ester of the formula

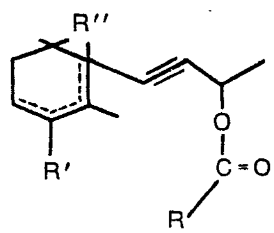

II

where R is a 1 to 6 carbon alkyl group, a phenyl radical or an alkylphenyl radical, the dotted lines represent a sole double bond in the 1- or 2- position or conjugated double bonds in the 1,3-position, and when the double bond is in the 1- position only, R′ is hydroxyl or

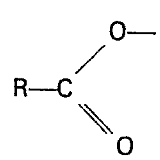

and R″ is absent, when the double bond is in the 2-position, R″ is hydroxyl and R′ is hydrogen, and when conjugated double bonds are present, R′ is hydrogen and R″ is absent with a catalytic amount of an essentially anhydrous mineral proton acid, organic proton acid, acidic diatomaceous earth or cation exchange resin in an organic solvent capable of forming an azeotrope with water.

The esters according to formula II are novel compounds represented by four species having the general formula III to VI

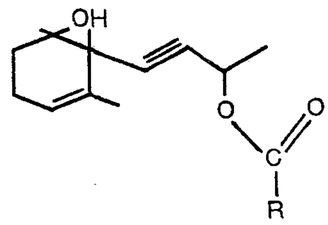

III

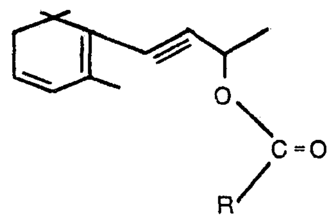

IV

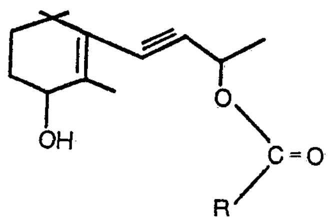

V

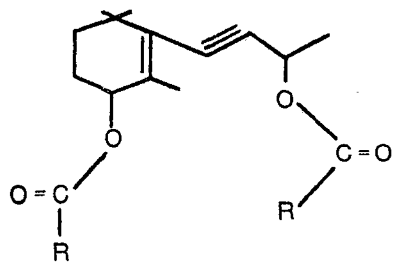

VI

The esters of formula III are prepared by direct acylation of the diol 2,6,6-trimethyl-1-hydroxy-1-(3-hydroxy-but-1-ynyl)cyclohex-2-ene. This diol is a known compound which can be prepared by reacting 2,6,6-trimethylcyclohex-2-ene-1-one with 3-hydroxy-but-1-yne in the presence of a lithium alkyl, a Grignard reagent, or metallic lithium and ammonia. See, e.g., Japanese patent application 74/75556 or British patent 1,335,339.

Acylation of the diol is carried out according to known methods such as, e.g., reacting the diol with the corresponding acid anhydride or acid halide under basic conditions. The formation of ester VI appears to take place via an allylic rearrangement of ester III when a basic environment is not provided during acylation.

Ester IV is a dehydration product prepared by acid anhydride treatment of the diol at room temperature and subsequent distillation in the presence of traces of acid.

Ester V is formed as a by-product resulting from an allylic rearrangement occurring during the preparation of ester III. It is also found as a product resulting from aqueous acid treatment of ester III at room temperature.

Specific examples of esters of formulae III to VI include, among others, the following:

2,6,6-trimethyl-1-hydroxy-1-(3-acetoxy-but-1-ynyl)cyclohex-2-ene
2,6,6-trimethyl-1-(3-acetoxy-but-1-ynyl)cyclohexa-1,3-diene
2,6,6-trimethyl-1-(3-benzoyloxy-but-1-ynyl)cyclohexa-1,3-diene
2,6,6-trimethyl-1-hydroxy-1-(3-benzoyloxy-but-1-ynyl)cyclohex-2-ene
2,6,6-trimethyl-3-hydroxy-1-(3-acetoxy-but-1-ynyl)cyclohex-1-ene
2,6,6-trimethyl-3-hydroxy-1-(3-benzoyloxy-but-1-ynyl)cyclohex-1-ene
2,6,6-trimethyl-3-acetoxy-1-(3-acetoxy-but-1-ynyl)cyclohex-1-ene
2,6,6-trimethyl-3-benzoyloxy-1-(3-benzoyloxy-but-1-ynyl)cyclohex-1-ene
2,6,6-trimethyl-1-hydroxy-1-(3-propionyloxy-but-1-ynyl)cyclohex-2-ene
2,6,6-trimethyl-1-(3-propionyloxy-but-1-ynyl)-cyclohexa-1,3-diene

The esters of formula III, V and VI are produced as mixtures of diastereoisomers from the corresponding diastereoisomeric diols. The isomer mixtures can be used as such in preparing the

desired 1-crotonoyl-2,6,6-trimethylcyclohexa-1,3-diene since the product ketone does not exhibit the same diastereoisomerism.

Conversion of the esters of formulae III to VI to 1-crotonoyl-2,6,6-trimethylcyclohexa-1,3-diene takes place smoothly and spontaneously by treating them with a catalytic amount of an essentially anhydrous proton acid. The reaction is carried out in an organic solvent which forms an azeotrope with the water generated by the reaction under temperature and reflux conditions such that the water is continuously removed.

Acids which can be employed in the reaction are either mineral acids such as phosphoric, nitric and sulfuric acid or organic carboxylic acids such as acetic acid, haloacetic acids, propionic acid, or sulfonic acids such as p-toluenesulfonic acid. Acidic agents such as acid cation exchange resins and acidic diatomaceous earth can also be used. Preferred acids are p-toluenesulfonic acid and phosphoric acid.

It is necessary that the acid be essentially anhydrous. In the context of this invention, essentially anhydrous means no more than about 15% by weight water. It is found that in acid of lower concentration, the yield of the desired 1-crotonoyl-2,6,6-trimethylcyclohexa-1,3-diene is adversely affected due to the presence of water.

Relatively small, catalytic amounts of the essentially anhydrous acid are required. About 0.2 to 5 mole % of the acid, based on the ester, is sufficient to accomplish the dehydration and deacylation.

As suggested hereinabove, the presence of water during the deacylation and dehydration steps is generally undesirable. In order to remove water from the reaction environment, the reaction is carried out in the presence of a solvent which forms an azeotrope with water at a relatively low temperature. Best results are obtained at temperatures between about 40 and 110°C., and preferably between 40 and 70°C. Solvents which form the necessary azeotrope within these temperature limits include, e.g., methylene chloride, chloroform, carbon tetrachloride, benzene, and toluene.

The invention is illustrated by the following examples.

Example 1

A. 2,6,6-Trimethyl-1-hydroxy-1-(3-acetoxy-but-1-ynyl)cyclohex-2-ene

In a 250 ml. three-necked flask fitted with a mechanical stirrer, a thermometer and a reflux condenser protected by a calcium chloride tube is stirred a mixture of 42 g. of 2,6,6-trimethyl-1-hydroxy-1-(3-hydroxy-but-1-ynyl)cyclohex-2-ene [prepared in accordance with the method described in Helv. Chim. Acta. *56*, 1503 (1973)] and 22 g. of acetic anhydride in 240 ml. of pyridine at ambient temperature for 24 hours. The reaction mixture is then poured into 240 ml. of water and extracted twice with pentane. The combined organic extracts are washed with 10% hydrochloric acid and then with 10% sodium bicarbonate solution and water, dried over sodium sulfate and concentrated to yield a residue which gives on distillation through a short Vigreux column 45 g. (89% yield) of a diastereoisomeric mixture of 2,6,6-trimethyl-1-hydroxy-1-(3-acetoxy-but-1-ynyl)cyclohex-2-ene, collected at 118°C./0.6 mm., $n_D^{21}$ 1.4910.

IR (neat): 3490, 3030, 2975, 2920, 2235, 1740, 1454, 1372, 1235, 1171, 1072, 1025, 995, 980, 967, 949, 834, 611 $cm^{-1}$.

NMR ($CDCl_3$): $\delta$1.04 (s, 6H), 1.48 (d, 3H), 1.86 (q, 1H), 2.05 (s, 3H), 2.29 (s, 1H), 5.46 (m, 2H) ppm.

Further distillation affords in ca. 5% yield, 2,6,6-trimethyl-3-hydroxy-1-(3-acetoxybut-1-ynyl)cyclohex-1-ene.

IR (neat): 3420, 2960, 2935, 2865, 2215, 1745, 1450, 1370, 1340, 1233, 1192, 1080, 1040, 1024, 965, 946, 903, 875, 845, 611, 522 $cm^{-1}$.

NMR ($CDCl_3$): $\delta$1.05 (s, 3H), 1.11 (s, 3H), 1.53 (d, 3H), 1.95 (s, 3H), 2.06 (s, 3H), 2.6 (OH), 3.42 (q, 1H), 3.93 (t, 1H), 6.54 (q, 1H) ppm.

B. 1-Crotonoyl-2,6,6-trimethylcyclohexa-1,3-diene

In a 500 ml. three-necked flask fitted with a mechanical stirrer and a Dean-Stark water separator provided with a reflux condenser is placed a solution of 40 g. of 2,6,6-trimethyl-1-hydroxy-1-(3-acetoxy-but-1-ynyl)cyclohex-2-ene in 250 ml. of methylene chloride. The solution is heated to gentle reflux and 1.3 g. of p-toluenesulfonic acid is added. The reaction mixture is refluxed for an additional 5 hours. The mixture is then cooled and poured into 250 ml. of water. The organic layer is separated, washed with 10% sodium bicarbonate solution, then with water and dried over sodium sulfate.

Concentration yields 40 g. of a residue which upon fractionation through a short Vigreux column gives 15 g. (50% yield) of the desired ketone, 1-crotonoyl-2,6,6-trimethylcyclohexa-1,3-diene, b.p. 75—80°C./0.1 mm., $n_D^{20}$ 1.5150.

IR (neat): 3040, 2960, 2915, 2890, 2715, 1670, 1645, 1613, 1460, 1441, 1396, 1378, 1357, 1303, 1291, 1250, 1222, 1180, 1156, 1057, 970, 930, 908, 845, 750, $\delta$697, 614, 553 $cm^{-1}$.

NMR ($CDCl_3$): 1.04 (s, 6H), 1.64 (s, 3H), 1.93 (dd, 3H), 5.80 (s, 2H), 6.18 (m, 1H), 6.80 (m, 1H) ppm.

## Example 2

A. 2,6,6-Trimethyl-1-(3-acetoxy-but-1-ynyl)cyclohexa-1,3-diene

In a 500 ml. three-necked flask fitted with a mechanical stirrer, a dropping funnel, a thermometer and a reflux condenser protected by a calcium chloride tube is placed a mixture of 66 g. of 2,6,6-trimethyl-1-hydroxy-1-(3-hydroxy-but-1-ynyl)cyclohex-2-ene and 76 g. of pyridine in 200 ml. of tertiary butyl methyl ether. To the stirred solution is added 34 g. of acetic anhydride at room temperature. The reaction mixture is stirred for a period of 27 hours at ambient temperature. The reaction mixture is then poured into 200 ml. of water and the organic layer is separated, washed with 10% hydrochloric acid, subsequently with water, 10% sodium bicarbonate solution and water, and finally dried over sodium sulfate. Concentration yields a residue which gives on distillation 33 g. or 45% yield of 2,6,6-trimethyl-1-(3-acetoxy-but-1-ynyl)cyclohexa-1,3-diene, b.p. 125°C./1 mm., $n_D^{20}$ 1.5045.

IR (neat): 3040, 2970, 2940, 2890, 2820, 2210, 1740, 1448, 1370, 1338, 1233, 1080, 1045, 1023, 946, 874, 835, 736, 706, 644, 609 $cm^{-1}$.

NMR ($CDCl_3$): $\delta$1.04 (s, 6H), 1.53 (d, 3H), 1.90 (s, 3H), 2.06 (s, 3H), 5.62 (q, 1H), 5.80 (2H) ppm.

B. 1-Crotonoyl-2,6,6-trimethylcyclohexa-1,3-diene

In a 100 ml. three-necked flask fitted with a mechanical stirrer and a Dean-Stark water separator provided with a reflux condenser, a solution of 5 g. of 2,6,6-trimethyl-1-(3-acetoxy-but-1-ynyl)cyclohexa-1,3-diene in 70 ml. of methylene chloride is refluxed while stirring. Then 0.2 g. of p-toluenesulfonic acid is added and the reaction mixture is refluxed for an additional 4 to 5 hours. The reaction mixture is cooled and poured into 70 ml. of water. The organic layer is separated, subsequently washed with 10% sodium bicarbonate solution and water, and finally dried over sodium sulfate. Concentration yields 5 g. of a residue which gives on distillation 2.5 g. or 60% yield of 1-crotonoyl-2,6,6-trimethylcyclohexa-1,3-diene, b.p. 75°—80°C./0.1 mm., $n_D^{20}$ 1.5150.

## Example 3

A. 2,6,6-Trimethyl-3-acetoxy-(3-acetoxy-but-1-ynyl)cyclohex-1-ene

In a 100 ml. three-necked flask fitted with a mechanical stirrer and a reflux condenser, a mixture of 10 g. of 2,6,6-trimethyl-1-hydroxy-1-(3-hydroxy-but-1-ynyl)cyclohex-2-ene and 20 g. of acetic anhydride is stirred at reflux temperature for 3 hours. Acetic acid and excess of acetic anhydride are distilled off under vacuo and the residue is distilled through a short Vigreux column. The product is collected as a diastereoisomeric mixture of 2,6,6-trimethyl-3-acetoxy-1-(3-acetoxy-but-1-ynyl)cyclohex-1-ene at 115°C./0.3 mm., $n_D^{20}$ 1.4910. It contains about 10% of 2,6,6-trimethyl-1-(3-acetoxy-but-1-ynyl)cyclohexa-1,3-diene. The yield is 9 g. or 64%.

IR (neat): 2960, 2935, 2865, 2215, 1740, 1446, 1370, 1340, 1230, 1148, 1080, 1043, 1019, 991, 961, 945, 870, 845, 606 $cm^{-1}$.

NMR ($CDCl_3$): $\delta$1.07 (s, 3H), 1.14 (s, 3H), 1.53 (d, 3H), 1.84 (s, 3H), 2.06 (s, 3H), 2.07 (s, 3H), 5.22 (t, 1H), 5.59 (q, 1H) ppm.

B. 1-Crotonoyl-2,6,6-trimethylcyclohexa-1,3-diene

In a 100 ml. three-necked flask fitted with a mechanical stirrer and a Dean-Stark water separator provided with a reflux condenser, a solution of 7 g. of 2,6,6-trimethyl-3-acetoxy-1-(3-acetoxy-but-1-ynyl)cyclohex-1-ene in 70 ml. of methylene chloride is refluxed while stirring. Then 0.7 g. of p-toluenesulfonic acid is added and the reaction mixture is refluxed for an additional 7 hours. The reaction mixture is cooled and then poured into 70 ml. of water. The organic layer is separated, washed with 10% sodium bicarbonate solution and water. Drying over sodium sulfate and subsequent concentration yields 5 g. of a residue which gives, upon distillation using a short Vigreux column, 3 g. or 60% yield of 1-crotonoyl-2,6,6-trimethylcyclohexa-1,3-diene, b.p. 75°—80°C./0.1 mm., $n_D^{20}$ 1.5150.

## Example 4

1-Crotonoyl-2,6,6-trimethylcyclohexa-1,3-diene

In a 2 l. three-necked flask fitted with a mechanical stirrer and a Dean-Stark water separator provided with a reflux condenser is placed a solution of 90 g. of crude 2,6,6-trimethyl-1-hydroxy-(3-benzoyloxy-but-1-ynyl)-cyclohex-2-ene [isolated from benzoylation of the corresponding diol, for the synthesis of the diol, see Helv. Chim. Acta *56*, 1503 (1973) and ibid. *56*, 1514 (1973)] in 600 ml. of alcohol free chloroform. Then 3 g. of p-toluenesulfonic acid is added and the reaction mixture is heated to reflux while stirring. Reflux is continued for 2 hours, while water is separated off. The reaction mixture is cooled and then poured into 600 ml. of water. The organic layer is separated, washed three times with 10% sodium bicarbonate solution to remove benzoic acid and then with water until neutral. Drying over sodium sulfate and subsequent concentration yields a residue which is flash-distilled. Redistillation using a short Vigreux column gives 22 g. or 40% yield of 1-crotonoyl-2,6,6-trimethylcyclohexa-1,3-diene, b.p. 75—80°C./0.1 mm., $n_D^{20}$ 1.5170.

**Claims**

1. A method of preparing 1-crotonoyl-2,6,6-trimethylcyclohexa-1,3-diene which comprises treating a compound having the general formula

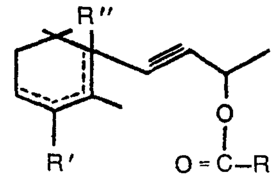

where R is a 1 to 6 carbon alkyl group, a phenyl radical or an alkylphenyl radical, the dotted lines represent a sole double bond in the 1- or 2-position or conjugated double bonds in the 1,3-position, and when the double bond is in the 1- position only, R′ is hydroxyl or

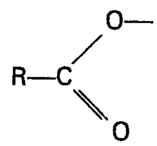

and R″ is absent, when the double bond is in the 2-position, R″ is hydroxyl and R′ is hydrogen, and when conjugated double bonds are present, R′ is hydrogen and R″ is absent with a catalytic amount of an essentially anhydrous mineral proton acid, organic proton acid, acidic diatomaceous earth or cation exchange resin in an organic solvent capable of forming an azeotrope with water.

2. The method of claim 1 wherein the proton acid is phosphoric acid or p-toluenesulfonic acid.

3. The method of claim 2 wherein the starting material has the formula

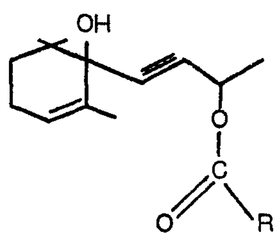

where R is a 1 to 6 carbon alkyl group, a phenyl radical or an alkylphenyl radical.

4. The method of claim 3 wherein the starting material is 2,6,6-trimethyl-1-hydroxy-1-(3-acetoxy-but-1-ynyl)cyclohex-2-ene and the proton acid reagent is p-toluenesulfonic acid.

5. The method of claim 3 wherein the starting material is 2,6,6-trimethyl-1-hydroxy-1-(3-benzoyloxy-but-1-ynyl)cyclohex-2-ene and the proton acid reagent is p-toluenesulfonic acid.

6. A chemical compound having the general structural formula

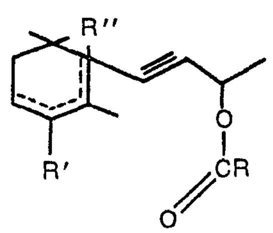

wherein R is a 1 to 6 carbon alkyl group, a phenyl radical or an alkylphenyl radical, the dotted lines represent a sole double bond in the 1- or 2-position or conjugated double bonds in the 1,3-position, and when the double bond is in the 1-position only, R′ is hydroxyl or

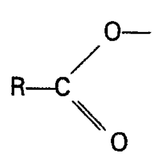

and R″ is absent, when the double bond is in the 2-position, R″ is hydroxyl and R′ is hydrogen, and when conjugated double bonds are present, R′ is hydrogen and R″ is absent.

7. 2,6,6-Trimethyl-1-hydroxy-1-(3-acetoxy-but-1-ynyl)cyclohex-2-ene.

8. 2,6,6-Trimethyl-1-hydroxy-1-(3-benzoyloxy-but-1-ynyl)cyclohex-2-ene.

9. 2,6,6-Trimethyl-3-acetoxy-1-(3-acetoxy-but-1-ynyl)cyclohex-1-ene.

10. 2,6,6-Trimethyl-3-benzoyloxy-1-(3-benzoyloxy-but-1-ynyl)cyclohex-1-ene.

**Patentansprüche**

1. Verfahren zur Herstellung von 1-Crotonoyl-2,6,6-Trimethylcyclohexa-1,3-dien wobei eine Verbindung der allgemeinen Formel

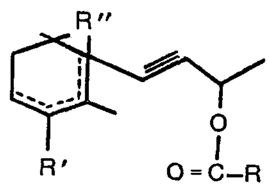

in welcher R eine $C_{1-6}$-Alkylgruppe, einen Phenylrest oder einen Alkylphenylrest, die gestrichelten Linien eine einzige Doppelbindung in 1- oder 2-Stellung oder konjugierte Doppelbindungen in 1,3-Stellung, und wenn die Doppelbindung nur in der 1-Stellung sich befindet R′ eine Hydroxylgruppe oder

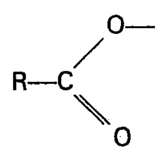

bedeuten und R″ abwesend ist, während wenn die Doppelbindung in 2-Stellung sich befindet, R″ Hydroxyl und R′ Wasserstoff bedeuten und wenn konjugierte Doppelbindungen anwesend sind, R′ Wasserstoff bedeutet und R″ abwesend ist, mit einer katalytischen Menge einer im wesentlichen wasserfreien mineralischen Protonensäure, organischen Protonensäure, sauren Diatomeenerde oder einem Kationen- Austauscherharz in einem organischen Lösungsmittel, welches in der Lage ist mit Wasser ein Azeotrop zu bilden, behandelt wird.

2. Verfahren gemäß Anspruch 1 worin die Protonsäure Phosphorsäure oder p-Toluolsulfonsäure ist. ist.

3. Verfahren gemäß Anspruch 2 worin das Ausgangsmaterial die Formel

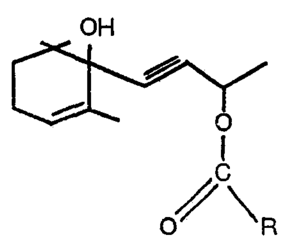

besitzt, worin R eine $C_{1-6}$-Alkylgruppe, einen Phenylrest oder einen Alkylphenylrest bedeutet.

4. Verfahren gemäß Anspruch 3 worin das Ausgangsmaterial 2,6,6-Trimethyl-1-hydroxy-1-(3-acetoxy-but-1-ynyl)-cyclohex-2-en und die Protonensäure p-Toluolsulfonsäure ist.

5. Verfahren gemäß Anspruch 3 worin das Ausgangsmaterial 2,6,6-Trimethyl-1-hydroxy-1-(3-benzoyloxy-but-1-ynyl)-cyclohex-2-en und die Protonensäure p-Toluolsulfonsäure ist.

6. Chemische Verbindung mit der allgemeinen Strukturformel

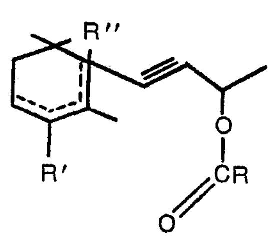

in welcher R eine $C_{1-6}$-Alkylgruppe, einen Phenylrest oder einen Alkylphenylrest, die gestrichelten Linien eine einzige Doppelbindung in 1- oder 2-Stellung oder konjugierte Doppelbindungen in 1,3-Stellung, und wenn die Doppelbindung nur in der 1-Stellung sich befindet R' eine Hydroxylgruppe oder

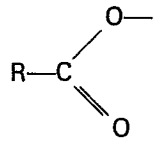

bedeuten und R'' abwesend ist, während wenn die Doppelbindung in 2-Stellung sich befindet, R'' Hydroxyl und R' Wasserstoff bedeuten und wenn konjugierte Doppelbindungen anwesend sind, R' Wasserstoff bedeutet und R'' abwesend ist.

7. 2,6,6-Trimethyl-1-hydroxy-1-(3-acetoxy-but-1-ynyl)-cyclohex-2-en.

8. 2,6,6-Trimethyl-1-hydroxy-1-(3-benzoyloxy-but-1-ynyl)cyclohex-2-en.

9. 2,6,6-Trimethyl-3-acetoxy-1-(3-acetoxy-but-1-ynyl)-cyclohex-1-en.

10. 2,6,6-Trimethyl-3-benzoyloxy-1-(3-benzoyloxy-but-1-ynyl) cyclohex-1-en.

**Revendications**

1. Procédé pour la préparation du 1-crotonyl-2,6,6-triméthylcyclohexa-1,3-diène caractérisé en ce que l'on traite un composé de formule

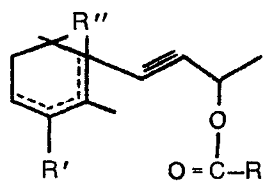

dans laquelle R est un groupe alkyle ayant de 1 à 6 atomes de carbone, un radical phényle, ou un radical alkylphényle, la ligne interrompue est une double liaison unique en position 1 ou 2 ou bien des doubles liaisons conjuguées en position 1, 3, et, exclusivement dans le cas où la double liaison se trouve dans la position ·1, R' est la fonction hydroxyle ou le groupe

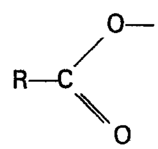

et R'' est absent, lorsque la double liaison se trouve dans la position 2, R'' est la fonction hydroxyle et R' est un atome d'hydrogène, et, lorsqu'il y a une paire de doubles liaisons conjuguées, R' est un atome d'hydrogène et R'' est absent, avec une quantité catalytique d'un acide protonique minéral sensiblement anhydre, d'un acide protonique organique, de terre de diatomées acide ou d'une résine échangeuse de cations, dans un solvant organique capable de former un azéotrope avec l'eau.

2. Procédé suivant la revendication 1 caractérisé en ce que l'acide protonique est l'acide phosphorique ou l'acide p-toluènesulfonique.

3. Procédé suivant la revendication 2 caractérisé en ce que le composé de départ répond à la formule

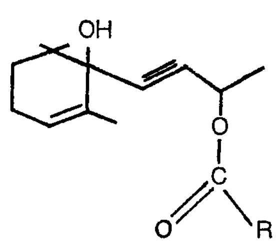

dans laquelle R est un groupe alkyle ayant de 1 à 6 atomes de carbone, un radical phényle ou un radical alkylphényle.

4. Procédé suivant la revendication 3 caractérisé en ce que le composé de départ est le 2,6,6-triméthyl-1-hydroxy-1-(3-acétoxy-but-1-ynyl)-cyclohex-2-ène et l'acide protonique est l'acide p-toluènesulfonique.

5. Procédé suivant la revendication 3 caractérisé en ce que le composé de départ est le 2,6,6-triméthyl-1-hydroxy-1-(3-benzoyloxy-but-1-ynyl)-cyclohex-2-ène et l'acide protonique est l'acide p-toluènesulfonique.

6. Produit chimique de formule générale

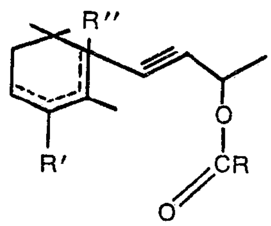

dans laquelle R est un groupe alkyle ayant de 1 à 6 atomes de carbone, un radical phényle, ou un radical alkylphényle, la ligne interrompue est une double liaison unique en position 1 ou 2 ou bien des doubles liaisons conjuguées en position 1, 3, et, exclusivement dans le cas où la double liaison se trouve dans la position 1, R' est la fonction hydroxyle ou le groupe

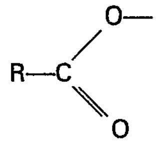

et R'' est absent, lorsque la double liaison se trouve dans la position 2, R'' est la fonction hydroxyle et R' est un atome d'hydrogène, et, lorsqu'il y a une paire de doubles liaisons conjuguées, R' est un atome d'hydrogène et R'' est absent.

7. 2,6,6-triméthyl-1-hydroxy-1-(3-acétoxy-but-1-ynyl)-cyclohex-2-ène.

8. 2,6,6-triméthyl-1-hydroxy-1-(3-benzoyloxy-but-1-ynyl)-cyclohex-2-ène.

9. 2,6,6-triméthyl-3-acétoxy-1-(3-acétoxy-but-1-ynyl)-cyclohex-1-ène.

10. 2,6,6-triméthyl-3-benzoyloxy-1-(3-benzoyloxy-but-1-ynyl)-cyclohex-1-ène.